# EUROPEAN PATENT SPECIFICATION

(11) **EP 2 974 595 B1**
(45) Date of publication and mention of the grant of the patent: **23.05.2018**
(21) Application number: 14764848.9
(22) Date of filing: 14.03.2014
(51) Int. Cl.: A01H 3/04, C07K 7/06

(54) **TREATING AGENT FOR PLANT CELL WALLS, SUBSTANCE DELIVERY METHOD USING TREATING AGENT, AND SUBSTANCE DELIVERY SYSTEM**
BEHANDLUNGSMITTEL FÜR PFLANZENZELLWÄNDE, SUBSTANZFREISETZUNGSVERFAHREN MIT DEM BEHANDLUNGSMITTEL UND SUBSTANZFREISETZUNGSSYSTEM
AGENT DE TRAITEMENT POUR PAROIS CELLULAIRES DE PLANTES, PROCÉDÉ D'ADMINISTRATION DE SUBSTANCE AU MOYEN DE L'AGENT DE TRAITEMENT, ET SYSTÈME D'ADMINISTRATION DE SUBSTANCE

(30) Priority: 15.03.2013 JP 2013053286
(43) Date of publication of application: 20.01.2016
(73) Proprietor: Suntory Holdings Limited, Osaka-shi, Osaka 530-8203 (JP)
(72) Inventor: MATSUI, Keisuke, Mishima-gun Osaka 618-8503 (JP)
(74) Representative: Vossius & Partner Patentanwälte Rechtsanwälte mbB
(86) International application number: PCT/JP2014/057937
(87) International publication number: WO 2014/142357

(56) References cited:
- WO-A1-00/05953
- WO-A1-94/00977
- WO-A1-03/101197
- WO-A1-2011/112570
- WO-A2-2011/046786
- WO-A2-2011/126644
- JP-A- H11 169 170
- JP-A- 2004 329 176
- JP-A- 2010 539 989
- EUDES FRANÇOIS ET AL: "Cell-penetrating peptides: From mammalian to plant cells", PLANT SIGNALING & BEHAVIOR, LANDES BIOSCIENCE, US, vol. 3, no. 8, 1 August 2008 (2008-08-01), pages 549-550, XP009135255, ISSN: 1559-2316

### Sequence Listing

### SEQUENCE LISTING

<110> SUNTORY HOLDINGS LIMITED
<120> Agent for Treating Cell Wall, Method for Delivering a Substance Using the Agent and a System for Delivering a Substance
<130> PCT13-0069
<150> JP 2013-053286
   <151> 2013-03-15
<160> 3
<170> PatentIn version 3.5
<210> 1
   <211> 9
   <212> PRT
   <213> Synthetic Construct
<400> 1
   Arg Lys Lys Arg Arg Gln Arg Arg Arg
   1 5
<210> 2
   <211> 9
   <212> PRT
   <213> Synthetic Construct
<400> 2
   Arg Arg Arg Arg Arg Arg Arg Arg Arg
   1 5
<210> 3
   <211> 15
   <212> PRT
   <213> Synthetic Construct
<400> 3
   Arg Arg Arg Arg Asn Arg Thr Arg Arg Asn Arg Arg Arg Val Arg
   1 5 10 15

## Description

### TECHNICAL FIELD

The present invention relates to a treating agent for plant cell walls, which comprises a chelator, a surfactant and a cell penetrating peptide and a substance delivery method using this treating agent. Herein also described are a whole plant transformed by this method, a processed product of this whole plant, as well as a substance delivery system comprising a chelator and a surfactant.

### BACKGROUND ART

Trait modification in plants is divided into two major types, i.e., variety improvement designed to make modifications even to their genetic traits, and transient trait modification caused by chemical agents or the like. Variety improvement intended to obtain plants with good traits is a process involving modifications of the genetic traits transmitted to the next and subsequent generations, and many attempts have been made repeatedly since a long time ago to obtain accidentally occurring mutants and to accomplish variety improvement by cross breeding, etc. These conventional breeding techniques have been used to create a large number of agricultural products and flowering plants showing traits useful for humans. However, these techniques have a problem in that a very long period of time is required to obtain desired traits, because these techniques rely on an insertion event of an accidental mutation which occurs with a low probability and/or on a process in which crossing should be repeated for several generations. Moreover, there have also been problems in that a trait which is not among the original genetic traits, such as a blue pigment in roses and carnations, has an extremely low probability of occurrence, and in that the degree and/or direction of mutation cannot be controlled as desired (Non-patent Document 1).

In recent years, variety improvement procedures based on new techniques have been introduced, as exemplified by mutation breeding through mutagen treatment using chemical agents or beam irradiation (e.g., radiation exposure) for increased probability of mutation, highly efficient cross breeding with the use of DNA markers, molecular breeding based on gene recombination technology, and so on (Non-patent Document 2). However, these new breeding procedures also have their pros and cons, as in the case of conventional breeding techniques. Even if the probability of mutation and/or the efficiency of crossing is increased, there remains a possibility that a desired trait cannot be obtained in the case of mutation breeding and cross breeding. Moreover, molecular breeding based on gene recombination technology is a very effective technique for obtaining a desired trait because this technique allows the use of various genetic resources from organisms of different species. However, due to the strict permit and approval system for genetically modified organisms (i.e., the "Act on the Conservation and Sustainable Use of Biological Diversity through Regulations on the Use of Living Modified Organisms" in Japan, which is commonly called the Cartagena Act) and a problem of public acceptance, even when plants with good traits can be successfully prepared, a long time is required until this technique becomes a regular practice under the present circumstances.

Transient trait modification includes techniques using plant hormones and/or chemical agents, e.g., for conversion into seedless fruits, induction of flowering, and improvement in the shelf life of cut flowers, and these techniques are also used widely in the agricultural field (Non-patent Document 3). However, the effects of plant hormones and/or chemical agents are often limited by plant species, and hence they will often have no effect on different plant species. Moreover, plant hormones and/or chemical agents are limited in type and therefore their effects are also few in kind; and hence it is difficult to say that they successfully meet various needs for trait modification.

On the other hand, with recent advances in molecular biology, findings have been obtained one after another showing that macromolecular compounds (e.g., RNAs, peptides, proteins) in plants are also transported through intercellular connection or sieve tubes to thereby control morphogenesis, flowering and other events (Non-patent Documents 4 to 6). If such a macromolecule which controls phenotypic expression in plants can be administered directly into a plant, it would be possible to achieve a simple and reliable technique for trait modification, in comparison with prior art techniques.

However, in the case of plant cells, two factors, i.e., their cell wall and cell membrane serve as barriers to transport of macromolecular compounds (e.g., proteins) from outside to inside of the cells (Non-patent Document 7).

### Non-patent Documents

Non-patent Document 1: G. Acquaah: Principles of Plant genetics and Breeding, Wiley-Blackwell (2012)
Non-patent Document 2: Patent Map for Each Technology Field, General 6, Variety improvement Techniques (1998)
Non-patent Document 3: L. Ostergaard: Annual Plant Reviews, Fruit Development and Seed Dispersal, Wiley-Blackwell (2010)
Non-patent Document 4: R. Ruiz-Medrano et al.: Curr. Opin. Cell Biol. 7, 641 (2004)
Non-patent Document 5: Y Ito et al.: Science 313, 842 (2006)
Non-patent Document 6: S. Tamaki et al.: Science 316, 1033 (2007)
Non-patent Document 7: F. Eudes and A. Chugh: Plant Signal. Behav. 3:8, 549 (2008)

### DISCLOSURE OF THE INVENTION

Under these circumstances, there is a need to develop a technique which allows macromolecular compounds to permeate through the two barriers of cell wall and cell membrane.

As a result of various efforts made to solve the problems stated above, the inventors of the present invention have found that when a solution containing a macromolecular compound in admixture with a cell penetrating peptide, a calcium chelator and a surfactant is prepared and directly applied to a plant, the macromolecular compound is efficiently taken up into cells. Moreover, the inventors of the present invention have also confirmed that when FT protein (Y. Kobayashi et al.: Science 286, 1960 (1999)), which is known as a promoter for flowering in whole plants, is applied to leaves, the introduced FT protein functions in the host plant to induce flower budding These findings led to the completion of the present invention.

The present invention is defined by the independent claims. Accordingly, the invention relates to a treating agent for plant cell walls, which allows introduction of a desired substance into plant cells, wherein said treating agent comprises a chelator, a surfactant and a cell penetrating peptide. Moreover, the invention is directed to a substance delivery method for introduction of a desired substance into a whole plant, which comprises the steps of:
(a) applying the treating agent according to any one of claims 1 to 4 to the surface of the whole plant; and
(b) applying the desired substance to the site where the treating agent has been applied, thereby introducing the desired substance into plant cells.
Preferred embodiments of the invention are defined by the dependent claims.

Herein described are also the following items:
[1] A treating agent for plant cell walls, which comprises a chelator and a surfactant.
[2] The treating agent according to [1] above, wherein the chelator is a calcium chelator.
[3] The treating agent according to [1] above, wherein the surfactant is a nonionic surfactant.
[4] A substance delivery method for introduction of a desired substance into a whole plant, which comprises the steps of:
   (a) applying the treating agent according to any one of [1] to [3] above to the surface of the whole plant; and
   (b) applying the desired substance to the site where the treating agent has been applied, thereby introducing the desired substance into plant cells.
[5] The method according to [4] above, wherein the desired substance is applied in admixture with a cell penetrating peptide.
[6] The method according to [4] above, wherein the desired substance has a molecular weight of 1 to 100 kDa.
[7] The method according to [6] above, wherein the desired substance is a macromolecule selected from the group consisting of sugars, proteins, nucleic acids and lipids.
[8] The method according to [5] above, wherein the cell penetrating peptide is an arginine-rich peptide.
[9] The method according to [4] above, wherein the steps (a) and (b) are carried out at the same time.
[10] A whole plant, a plant organ, a plant tissue, a plant cell, a protoplast, a leaf section or a callus, which comprises a desired substance introduced by the method according to any one of [4] to [9] above.
[11] A substance delivery system for introduction of a desired substance into a whole plant, which comprises a chelator and a surfactant.
[12] The system according to [11] above, which further comprises a cell penetrating peptide.
[13] The system according to [11] above, wherein the chelator is a calcium chelator.
[14] The system according to [11] above, wherein the surfactant is a nonionic surfactant.
[15] The system according to [12] above, wherein the cell penetrating peptide is an arginine-rich peptide.

The method of the present invention can cause phenotypic changes in host plants without changing their genetic traits. Moreover, because of being free from any gene recombination step, the method of the present invention can cause phenotypic changes in host plants in a simple manner.

### BRIEF DESCRIPTION OF THE DRAWINGS

Figure 1 shows protoplasts of torenia, into which GFP was introduced using a cell penetrating peptide (BF: bright field, GFP: GFP fluorescence).
Figure 2 shows epidermal cells of onion bulbs, into which GFP or GFP-FT was introduced using a cell penetrating peptide (BF: bright field, GFP: GFP fluorescence).
Figure 3 shows the results obtained for FT protein introduced into whole plants through their leaves by the method of the present invention, as detected by Western blotting in their shoot apex tissues.
Figure 4 shows the flowering state of whole plants, into which FT protein was introduced by the method of the present invention.

### BEST MODES FOR CARRYING OUT THE INVENTION

The present invention will be described in more detail below. The following embodiments are illustrated to describe the present invention. The present invention can be implemented in various modes, without departing from the scope of the present invention which is defined by the independent claims.

### 1. Treating agent for plant cell walls

In general, the cell walls of whole plants have a network structure mainly composed of cellulose and hemicellulose, etc., and spaces in the network are filled with pectin, glycoproteins and so on. For this reason, the pore size of cell walls is considered to be around 2 nm although it will vary depending on plant species so that the diffusion of water molecules is also considered to be nearly 10- to 100-fold slower within the cell walls (E. Kramer et al.: J. Exp. Bot. 58(11), 3005 (2007)).

Thus, in cases where whole plants are administered with a certain substance (hereinafter referred to as a "desired substance") in an attempt to modify their traits (e.g., to impart environmental resistance), a substance having a molecular size greater than the pore size of cell walls will not be taken up into plant cells because the presence of cell walls serves as an obstacle.

For example, macromolecules such as proteins have a diameter of 5 nm or more, even in their folded state, and therefore cannot permeate through cell walls (whose pore size is about 2 nm).

To increase the permeability of cell walls, there is a need to loosen the binding of their constituents, i.e., cellulose, hemicellulose, xylose, xyloglucan, pectin and so on. It is common practice to extract cell wall components by treatment with cell wall lyases (e.g., cellulase) or with organic solvents (e.g., acetone) (T. A. Gorshkova et al.: Plant Physiol. 110, 721 (1996)), but there are few reports showing that living cells are directly treated to increase the permeability of their cell walls.

To solve this technical problem, the present invention provides a treating agent for plant cell walls, which comprises a chelator, a surfactant and a cell penetrating peptide. When applied to a whole plant, the treating agent of the present invention allows a desired substance to be introduced into plant cells.

In the context of the present invention, the "chelator" is not limited in any way as long as it has the ability to chelate metal molecules or metal ions (e.g., calcium, potassium, sodium, magnesium, aluminum, iron, zinc) present within plant cell walls, but it is preferably a divalent metal chelator, and more preferably a calcium chelator.

Specific examples of chelators available for use in the present invention include, but are not limited to, 2,2'-bipyridine, 4,4'-bipyridine, ethylenediamine (EDA), diethylenetriamine (DETA), triethylenetetramine (TETA), tetraethylenepentamine (TEPA), pentaethylenehexamine (PEHA), phenanthroline, crown ethers (12-crown-4, 15-crown-5, 18-crown-6, dibenzo-18-crown-6), azacrown ethers (e.g., diaza-18-crown-6, 1,4,7,10-tetraazacyclododecane), ethylenediaminetetraacetic acid (EDTA), ethylenediamine-*N*,*N'*-disuccinic acid (EDDS), 1,4,7,10-tetraazacyclododecane-1,4,7,10-tetraacetic acid (DOTA), iminodiacetic acid (IDA), 1-hydroxyethane-1,1-diphosphonic acid (HEDP), *O,O'*-bis(2-aminophenyl)ethylene glycol*-N,N,N',N'-*tetraacetic acid (BAPTA), *O,O'*-bis(2-aminoethyl)ethylene glycol-*N,N,N',N'*-tetraacetic acid (EGTA), trans-1,2-diaminocyclohexane-*N,N,N',N'*-tetraacetic acid (CDTA) and so on.

The most preferred chelators for use in the present invention are EDTA and EGTA.

The treating agent of the present invention comprises such a chelator as listed above at a concentration of 10 µM to 500 mM, 50 µM to 450 mM, 100 µM to 400 mM, 500 µM to 350 mM, 1 mM to 300 mM, 5 mM to 250 mM, 10 mM to 200 mM, 15 mM to 150 mM, 20 mM to 100 mM, 20 mM to 50 mM, 20 mM to 30 mM, 1 to 25 mM, or 1 mM.

The "surfactant" available for use in the present invention is not limited in any way as long as it has the ability to form micelles in a solution, and it may be any of an anionic surfactant, a cationic surfactant, an amphoteric surfactant or a nonionic surfactant. Preferred surfactants for use in the present invention are nonionic surfactants, more preferably nonionic surfactants whose ability to solubilize membranes is weak, and even more preferably surfactants containing a polyether-modified silicone. Particularly preferred are surfactants for use in transformation.

Specific examples of such surfactants include, but are not limited to, a polyether-modified silicone (trisiloxane ethoxylate) (Silwet L-77), polyoxyethylene sorbitan monooleate (Tween 80), polyoxyethylene sorbitan monolaurate (Tween 20), octylphenol poly(ethylene glycol ether) (Triton X-100) and so on.

The most preferred surfactants for use in the present invention are Silwet L-77 and Tween 20.

The treating agent of the present invention comprises such a surfactant as listed above at a concentration of 0.0001% to 50%, 0.0005% to 25%, 0.001% to 10%, 0.001% to 5%, 0.001% to 1%, 0.001% to 0.1%, 0.001% to 0.05%, 0.001% to 0.01%, or 0.001% to 0.005%.

The "treating agent" of the present invention is a chemical agent for increasing the cell wall permeability of a desired substance.

When the treating agent of the present invention is applied to a whole plant or a part thereof to treat its cell walls, a desired substance can be administered to the whole plant via the treated site, thereby resulting in an increased percentage and/or rate of cell wall permeation of the desired substance, when compared to no application.

Such a desired substance is not limited in any way and may be either a small molecular compound or a macromolecule selected from the group consisting of sugars, proteins, nucleic acids and lipids.

In the context of the present invention, the desired substance is preferably a compound having a molecular weight of 0.01 to 1000 kDa, 0.05 to 750 kDa, 0.1 to 500 kDa, 0.5 to 250 kDa, 1 to 100 kDa, 5 to 75 kDa, 10 to 50 kDa, or 15 to 25 kDa.

Specific examples of such a desired substance include compounds for promoting the flowering of whole plants, compounds for controlling the morphology of plants, compounds for changing the scent of flowers, substances for changing the taste of fruits, pigment-related compounds, environmental resistance factors and so on.

Compounds for promoting the flowering of whole plants may be exemplified by FT protein (FLOWERING LOCUS T protein) and a gene encoding this protein (Y. Kobayashi et al.: Science 286, 1960 (1999)), as well as geranyl pyrophosphate synthase large subunit (Japanese Patent Application No. 2012-149409), etc.

Compounds for controlling the morphology of plants may be exemplified by a group of genes (MIDD1, ROP11, ROPGAP3, ROPGEF4) each controlling the construction of plant cell walls (Y. Oda and H. Fukuda, Science 337, 1333(2012)), as well as a group of genes (ACE, PRE1, AtIBH1) each regulating the length of cells (M. Ikeda et al.: The Plant Cell 24, 4483(2012)), etc.

Compounds for enhancing the scent of flowers may be exemplified by monoterpene glycosyltransferase (Japanese Patent Application No. 2012-022984, Japanese Patent Application No. 2012-022982, Japanese Patent Application No. 2012-022983, Japanese Patent Application No. 2012-022985, PCT/JP2011/080584) and so on.

Compounds for changing the taste of fruits may be exemplified by steviol synthase (Japanese Patent Application No. 2012-060473, Japanese Patent Application No. 2012-071959), while pigment-related compounds may be exemplified by anthocyanidin synthase (D. Weiss et al.: Plant Mol. Biol. 22, 893(1993)) and flavonol hydroxylase (Anzellotti D. and Ibrahim R. K., 2004. BMC Plant Biol. 4, 20; Halbwirth H. et al., 2004. Plant Sci. 167, 129-135; PCT/JP2011/068683), etc.

Fatty acid synthases may be exemplified by acyl-CoA synthase (PCT/JP2011/052035), glycerol-3-phosphate acyltransferase (PCT/JP2010/066280), diacylglycerol acyltransferase (PCT/JP2010/072930), fatty acid elongase (PCT/JP2012/069792) and so on.

Further, environmental resistance factors may be exemplified by compounds for changing the susceptibility and/or resistance of host plants to temperature, light intensity, salt concentration, drying conditions, radiation dose, and others.

The target to which the treating agent of the present invention is applied may be any of a whole plant, a plant organ (e.g., leaf, petal, stem, root, seed), a plant tissue (e.g., epidermis, phloem, parenchyma, xylem, vascular bundle, palisade tissue, spongy parenchyma) or a plant cultured cell, or alternatively, various forms of plant cells (e.g., suspended cultured cells), a protoplast, a leaf section, a callus and so on. The plant used for transformation may be of any type, belonging to either monocotyledons or dicotyledons.

A desired substance may be administered in any manner, e.g., by using a cell penetrating peptide, by liposome-mediated techniques, by electroporation techniques (competent cells, protoplasts), etc. When applying a treating agent comprising a chelator and a surfactant, the introduction efficiency of a desired substance is increased even in the case of conventionally used techniques for substance introduction (e.g., gene transfer techniques such as Agrobacterium-mediated techniques, bombardment techniques, plant virus vector-mediated techniques and so on).

In the context of the present invention it has been found that a desired substance can be administered with a particular high efficacy using a cell penetrating peptide. The use of a cell penetrating peptide allows simple introduction of a desired substance, regardless of its type, i.e., sugar, protein, nucleic acid or lipid.

Cell penetrating peptides (CPPs) refer to a group of arginine-rich peptides found from among peptides which are used by viruses during their infection. Cell penetrating peptides are known to cause micropinocytosis, a kind of endocytosis, on the cell membrane, as a result of which macromolecules located around the cell membrane are taken up into cells (A. Chugh et al.: Life 62, 183 (2010)). Virus-derived cell penetrating peptides of various sequences are known and are regarded as being effective for intracellular uptake of macromolecular compounds in animal cells. Moreover, it is known that not only virus-derived sequences, but also peptides composed of about 10 arginine residues simply connected one after another have the same effect, and the R9 peptide (i.e., a peptide composed of 9 arginine residues) has been reported in tobacco cultured cells to act on protein uptake into plant cells (M. Mae et al.: BBA 1669, 101 (2005)).

Accordingly, the treating agent of the present invention comprises a CPP in addition to a chelator and a surfactant.

Preferably, the CPP for use in the present invention is a peptide having a sequence length of 5 to 50 aa, 5 to 40 aa, 5 to 30 aa, 5 to 20 aa, or 5 to 15 aa.

Preferably, the CPP for use in the present invention is an arginine-rich peptide (i.e., a peptide in which the number of arginine residues in the entire amino acid sequence is 60% or more, 70% or more, 80% or more, 90% or more, or 95% or more).

There are several types of CPPs, and it is possible to use HIV Tat (transactivating protein)-derived CPP (SEQ ID NO: 1), R9 CPP (SEQ ID NO: 2) or FHV-derived CPP (SEQ ID NO: 3) in the present invention.

The most preferred CPP for use in the present invention is a peptide consisting of an amino acid sequence sharing a sequence identity of 70% or more, 80% or more, or 90% or more with the amino acid sequence of SEQ ID NO: 2.

In the present invention, a desired substance may be administered in a state of being linked to a CPP, or alternatively, may be administered as a mixture obtained by simply mixing the desired substance and a CPP, without being linked to the CPP.

In cases where a desired substance and a CPP are mixed to prepare a mixture, the molar ratio between the desired substance and the CPP is 1:1 to 1:10, 1:1 to 1:60, 1:1 to 1:100, 1:1 to 1:1000, 1:5 to 1:10, 1:5 to 1:100, or 1:5 to 1:1000.

For details of CPP-mediated transformation techniques and their conditions, reference may be made to the method of Langel et al. (M. Mae et al.: Biochim. Biophys. Acta 1669, 101 (2005), S. El-Andaloussi et al.: Biochem. J. 407, 285 (2007)).

### 2. Delivery method

As a second embodiment, the present invention provides a substance delivery method using the treating agent of the present invention. Namely, the present invention provides a substance delivery method for introduction of a desired substance into a whole plant, which comprises the steps of:
(a) applying the treating agent of the present invention to the surface of the whole plant; and
(b) applying the desired substance to the site where the treating agent has been applied, thereby introducing the desired substance into plant cells.

In the step (a), the treating agent of the present invention is applied to the surface of a whole plant. This may be accomplished by bringing a solution containing the treating agent of the present invention into contact with the surface of the above whole plant or a tissue thereof by means of a brush, a cloth, a dropper, a pipet, a capillary tube or the like. Alternatively, a solution containing the treating agent of the present invention may be applied by being sprayed on the above whole plant through an atomizer or the like.

Application should not always be conducted throughout the whole plant, as already described above.

In the step (b), techniques as already described above may be used to apply a desired substance to the site where the treating agent has been applied, thereby introducing the desired substance into plant cells. Thus, the step (b) may preferably be accomplished by using a cell penetrating protein.

In this case, a desired substance may be applied in a state of being linked to a CPP, or alternatively, may be applied as a mixture obtained by simply mixing the desired substance and a CPP, without being linked to the CPP.

In the delivery method of the present invention, the steps (a) and (b) may be carried out at a certain interval (1 hour, 6 hours, 12 hours, 24 hours, 48 hours, 72 hours) or may be carried out at the same time.

When the steps (a) and (b) are carried out at the same time, a possible method for this purpose involves mixing the treating agent of the present invention with a desired substance prior to application to a whole plant, and applying the resulting mixture to the whole plant.

Alternatively, the treating agent of the present invention comprising a CPP may be mixed with a desired substance and the resulting mixture may be applied to a whole plant.

Moreover, in the method of the present invention, the steps (a) and (b) may be repeated depending on the type of whole plant. For example, the above steps (a) and (b) may each be repeated twice, three times, four times or five times. Even when repeated, the steps (a) and (b) may be carried out at the same time.

Whether or not a desired substance has been introduced may be confirmed by collecting a tissue of the above whole plant and analyzing the tissue or an extract thereof by *in situ* hybridization, immunohistological staining, Northern blotting, Southern blotting, Western blotting, RT-PCR, ELISA, etc.

Alternatively, whether or not a desired substance has been introduced may also be confirmed by the presence or absence of a phenotype resulting from the desired substance (e.g., a change in flowering time, a change in petal color, a change in scent, a change in environmental resistance).

### 3. Whole plants

Herein also described is a transgenic whole plant transformed by the delivery method of the present invention.

Such transgenic plants include plants of the family Solanaceae (e.g., eggplant, tomato, hot pepper, potato, tobacco, stramonium, Chinese lantern plant, petunia, calibrachoa, nierembergia), plants of the family Leguminosae (e.g., soybean, adzuki bean, peanut, kidney bean, broad bean, Bird's foot trefoil), plants of the family Rosaceae (e.g., strawberry, Japanese apricot, cherry tree, rose, blueberry, blackberry, bilberry, cassis, raspberry), plants of the family Caryophyllaceae (e.g., carnation, gypsophila), plants of the family Asteraceae (e.g., chrysanthemum, gerbera, sunflower, daisy), plants of the family Orchidaceae (e.g., orchid), plants of the family Primulaceae (e.g., cyclamen), plants of the family Gentianaceae (e.g., showy prairie gentian, gentian), plants of the family Iridaceae (e.g., freesia, iris, gladiolus), plants of the family Scrophulariaceae (e.g., snapdragon, torenia), plants of the family Crassulaceae (e.g., kalanchoe), plants of the family Liliaceae (e.g., lily, tulip), plants of the family Convolvulaceae (e.g., morning glory, ivy-leaved morning glory, moonflower, sweet potato, cypress vine, evolvulus), plants of the family Hydrangeaceae (e.g., hydrangea, deutzia), plants of the family Cucurbitaceae (e.g., bottle gourd), plants of the family Geraniaceae (e.g., pelargonium, geranium), plants of the family Oleaceae (e.g., weeping forsythia), plants of the family Vitaceae (e.g., grape), plants of the family Theaceae (e.g., camellia, tea plant), plants of the family Gramineae (e.g., rice, barley, wheat, oat, rye, maize, foxtail millet, Japanese barnyard millet, kaoliang, sugar cane, bamboo, wild oat, finger millet, sorghum, Manchurian wild rice, job's tears, pasture grass), plants of the family Moraceae (e.g., mulberry, hop, paper mulberry, rubber tree, cannabis), plants of the family Rubiaceae (e.g., coffee tree, gardenia), plants of the family Fagaceae (e.g., oak, beech, Japanese emperor oak), plants of the family Pedaliaceae (e.g., sesame), plants of the family Rutaceae (e.g., bitter orange, Citrus junos, satsuma mandarin, Japanese pepper tree), and plants of the family Brassicaceae (e.g., red cabbage, flowering cabbage, Japanese radish, white shepherd's purse, Chinese colza, cabbage, broccoli, cauliflower).

Examples of preferred plants include ornamental plants, as exemplified by plants of the family Caryophyllaceae (e.g., carnation, gypsophila), plants of the family Scrophulariaceae (e.g., snapdragon, torenia), plants of the family Solanaceae (e.g., eggplant, tomato, hot pepper, potato, tobacco, stramonium, Chinese lantern plant, petunia, calibrachoa, nierembergia), plants of the family Leguminosae (e.g., soybean, adzuki bean, peanut, kidney bean, broad bean, Bird's foot trefoil), plants of the family Rosaceae (e.g., strawberry, Japanese apricot, cherry tree, rose, blueberry, blackberry, bilberry, cassis, raspberry), plants of the family Asteraceae (e.g., chrysanthemum, gerbera, sunflower, daisy), plants of the family Orchidaceae (e.g., orchid), plants of the family Primulaceae (e.g., cyclamen), plants of the family Gentianaceae (e.g., showy prairie gentian, gentian), plants of the family Iridaceae (e.g., freesia, iris, gladiolus), plants of the family Crassulaceae (e.g., kalanchoe), plants of the family Liliaceae (e.g., lily, tulip), plants of the family Convolvulaceae (e.g., morning glory, ivy-leaved morning glory, moonflower, sweet potato, cypress vine, evolvulus), plants of the family Cucurbitaceae (e.g., bottle gourd), plants of the family Hydrangeaceae (e.g., hydrangea, deutzia), plants of the family Geraniaceae (e.g., pelargonium, geranium), plants of the family Oleaceae (e.g., weeping forsythia), and plants of the family Theaceae (e.g., camellia, tea plant), as well as plants whose fruits or seeds obtained after flowering are also useful, as exemplified by plants of the family Gramineae (e.g., rice, barley, wheat, oat, rye, maize, foxtail millet, Japanese barnyard millet, kaoliang, sugar cane, bamboo, wild oat, finger millet, sorghum, Manchurian wild rice, job's tears, pasture grass), plants of the family Moraceae (e.g., mulberry, hop, paper mulberry, rubber tree, cannabis), plants of the family Rubiaceae (e.g., coffee tree, gardenia), plants of the family Pedaliaceae (e.g., sesame), plants of the family Rutaceae (e.g., bitter orange, Citrus junos, satsuma mandarin, Japanese pepper tree), plants of the family Vitaceae (e.g., grape), and plants of the family Brassicaceae (e.g., Japanese radish, white shepherd's purse, Chinese colza), etc.

Plants prepared by the method of the present invention may be provided for appreciation or sale in any state selected from soil-grown plants, potted plants, cut flowers or flowers alone. Furthermore, portions of flowers, e.g., corolla, petals or calyxes alone may also be provided for appreciation or sale. Likewise, the plant of the present invention may also be provided for sale in the form of fruits or seeds.

Moreover, the plant prepared by the method of the present invention can be easily obtained as a perfect whole plant by being grown from a seed, a cuttage, a bulb or the like of the plant.

Thus, the plant prepared by the method of the present invention encompasses a whole plant, a plant organ (e.g., leaf, petal, stem, root, seed, bulb), a plant tissue (e.g., epidermis, phloem, parenchyma, xylem, vascular bundle, palisade tissue, spongy parenchyma) or a plant cultured cell, or alternatively, various forms of plant cells (e.g., suspended cultured cells), a protoplast, a leaf section, a callus and so on.

If the host plant transformed by the delivery method of the present invention is a portion of a whole plant, such as a plant organ, a plant tissue, a plant cell, a protoplast, a leaf section or a callus, the resulting transformant may be grown in an appropriate environment until a perfect whole plant is formed. With regard to techniques for growing a portion of a whole plant into a perfect whole plant, reference may be made to the descriptions in the following document: Biochemistry Experiments Vol. 41, An Introduction to Plant Cell Technology, Japan Scientific Societies Press, ISBN 4-7622-1899-5.

### 4. Processed products derived from the plant prepared by the method of the present invention

Today, not only natural flowers (e.g., soil-grown plants, potted plants, cut flowers, tissue culture shoots), but also processed products of natural flowers are sold as products for plant appreciation. A whole plant prepared by the method of the present invention is also very useful as a material for such processed products of natural flowers. Thus, herein also described is a processed product derived from the plant prepared by the method of the present invention (e.g., natural flower, cut flower) or a portion thereof (e.g., leaf, petal, stem, root, seed, bulb). Examples of such a processed product include, but are not limited to, pressed flowers, dried flowers, preserved flowers, material flowers, resin-embedded products, etc.

### 5. Substance delivery system

Herein also described is a substance delivery system for introduction of a desired substance into a whole plant. This system comprises the above chelator and surfactant.

Further, this system may comprise a cell penetrating peptide in addition to the above chelator and surfactant.

The chelator, surfactant and cell penetrating peptide contained in the substance delivery system are as already described above.

The use of the substance delivery system allows simple introduction of a desired substance into a whole plant to thereby cause a change in the traits of the whole plant.

### EXAMPLES

The present invention will be described in more detail below by way of the following illustrative examples, although the present invention is not limited to the embodiments disclosed herein.

### [Example 1] Verification of cell penetrating peptide R9 using plant cells

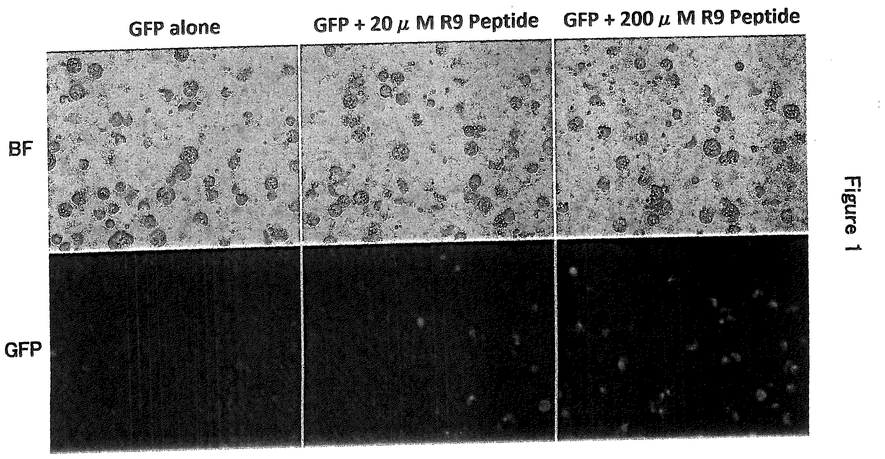

To verify that the R9 peptide was also evenly effective for plant cells other than cultured cells, protoplasts of torenia were used and observed for their uptake of green fluorescent protein (GFP) in the presence of the R9 peptide. Torenia leaves (0.5 g) were treated in an enzyme solution (1% Onozuka RS, 0.1% pectolyase Y-23, 0.45 M mannitol) at 30°C for 2 hours to prepare protoplasts. The protoplasts were washed with 0.45 M mannitol and then divided into 50 µl aliquots, followed by centrifugation to prepare protoplasts. The protoplasts were each suspended in 50 µl of a buffer (0.45 M mannitol, 1 × PBS, 0.1 mg GFP, (0, 20, 200 µM) R9 peptide) and observed for intracellular migration of GFP under a fluorescence microscope (Figure 1).

With increase in the concentration of the R9 peptide, cells showing GFP uptake were confirmed to increase in number. In the presence of 200 µM R9 peptide, good GFP uptake was observed, thus confirming that the R9 peptide was also effective for uptake through the cell membrane in plant cells.

### [Example 2] Permeability of cell walls

Various test solutions containing a fluorescent protein, GFP or GFP-FT, and the R9 peptide were prepared and applied to onion bulbs for verification of GFP uptake. The ingredients of the test solutions verified are shown below:
salt (NaCI), acidic buffer (citric acid), organic solvent (toluene, ethanol), cell wall lyase (pectolyase), surfactant (saponin, Silwet L-77, Tween 20, Triton X-100, CHAPS, CTAB, SDS), chelator (EGTA, EDTA, CDTA, BAPTA), DMSO, and glycerol.

The ingredients listed above were used alone or in combination to repeat trial and error experiments. As a result, it was found that the cell wall permeability in living cells could be increased upon combination of a calcium chelator (EDTA, EGTA) and a surfactant (Silwet L-77, Tween 20).

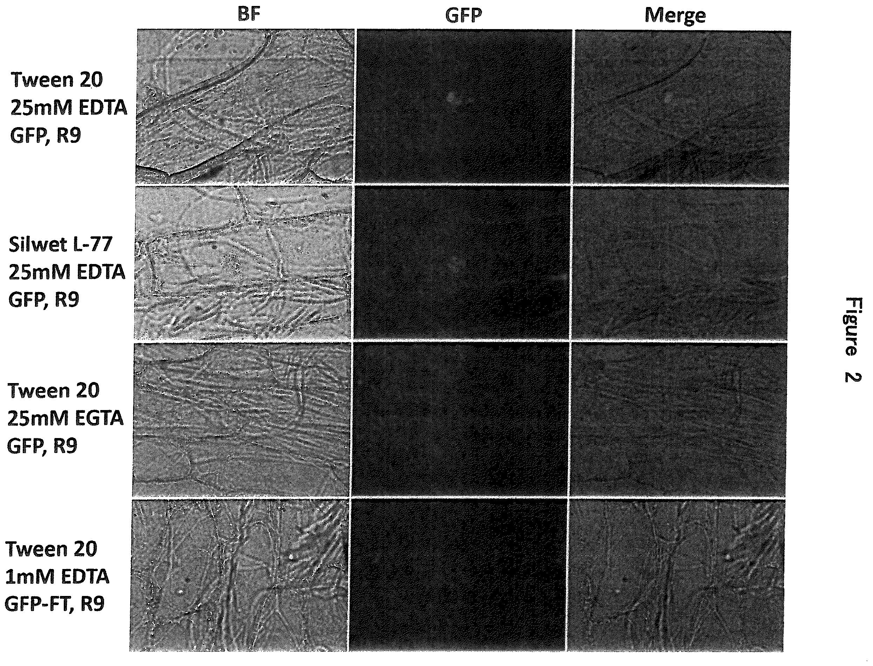

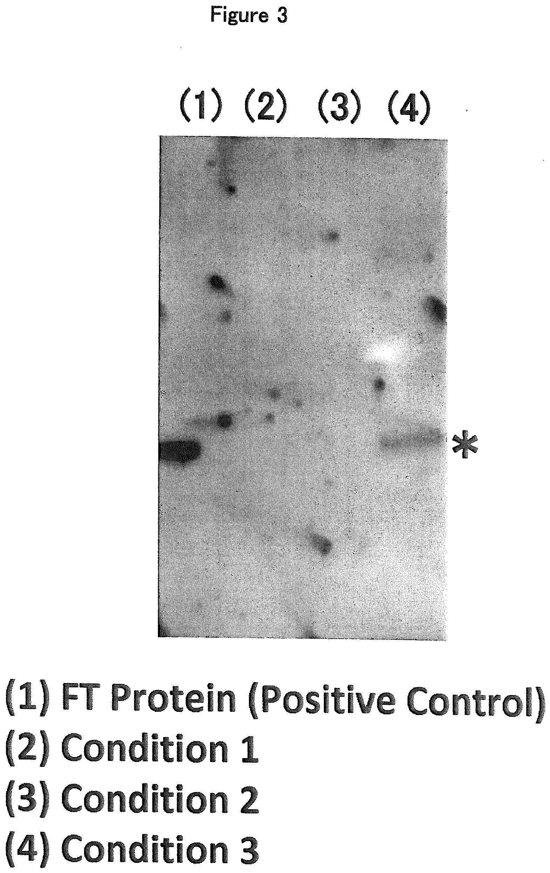

Onion bulbs were cut into boat-shaped pieces, and test solutions (100 µl each) prepared at various concentration conditions (0.1 × PBS, 1 to 100 mM chelator, 0.005% to 0.05% surfactant, 0.1 mg GFP, 20 µM R9 peptide) were applied to their surface. Each sample was allowed to stand in a dish at room temperature for 12 hours while being kept under humid conditions. Then, the test solutions were removed and the samples were washed with distilled water. After a 0.25% trypsin solution was applied in 100 µl volumes, the samples were allowed to stand for 30 minutes. After removal of the trypsin solution, the samples were washed with distilled water and their epidermal cell layers were peeled to prepare specimens, which were then observed for intracellular uptake of GFP under a fluorescence microscope (Leica, DM6000B). In the case of a chelator-free and surfactant-free test solution, strong fluorescence was observed on the cell wall surface before trypsin treatment, but the fluorescence disappeared after trypsin treatment and no fluorescence was observed within the cells. This finding suggested that a mixture of GFP and the R9 peptide would not be able to permeate through cell walls and hence GFP would be adhered onto the cell wall surface. Moreover, this strong adhesion onto the cell wall surface was not observed upon treatment with GFP alone, thus suggesting that GFP and the R9 peptide would form a highly adhesive complex. On the other hand, in samples receiving application of test solutions containing 1 to 50 mM EDTA or EGTA and 0.005% to 0.01% Silwet L-77 or Tween 20, granular GFP fluorescence was strongly observed around the nuclei of onion epidermal cells (Figure 2). This finding suggested that upon combination of EDTA or EGTA serving as a chelator and Silwet L-77 or Tween 20 serving as a surfactant, it would be possible to increase the permeability of cell walls, as a result of which GFP would be taken up into the cells. Further, when a fusion protein (molecular weight: about 50 kDa) formed between GFP and FT protein was used instead of GFP under conditions containing a chelator and a surfactant, this fusion protein was confirmed to be taken up into cells (Figure 3). This result suggests that the combination of a chelator and a surfactant would increase the permeability of cell walls to thereby allow permeation of substances whose size is greater than the maximum pore size of cell walls, i.e., 25 kDa (N. Capita et al.: Science 205, 1141 (1979)).

### [Example 3] Verification of macromolecular compound (FT protein) migration

The FT gene was first identified as one of the responsible genes for late flowering mutants and was suggested to be among the major regulatory factors for day length sensitivity, together with CO and GI genes (M. Koornneef et al.: Mol. Gen. Genet. 229, 57 (1991)). Thereafter, the FT gene was sequenced and FT-overexpressing plants were analyzed, thus indicating that early flowering traits would be acquired simply upon overexpression of the FT gene (Y. Kobayashi et al.: Science 286, 1960 (1999)). Moreover, experiments using grafts and analysis of expression sites suggested that the FT protein was a protein biosynthesized in leaves and transported to the shoot apex, and would be a possible candidate for florigen (H. An et al.: Development 131, 3615 (2004)). The identity of florigen, which remained unknown for many years, was elucidated in 2007 and shown to be the FT protein (rice Hd3a protein) (L. Corbesier et al.: Science 316, 1030 (2007), S. Tamaki et al.: Science 316, 1033 (2007)). Since the FT protein is a protein having the property of migrating from leaves to the shoot apex, if the FT protein applied to leaves can be confirmed to have migrated to the shoot apex, such confirmation would serve as a proof of uptake techniques of macromolecular compounds from the extracellular environment.

To verify the migration of the FT protein applied to leaves, the shoot apex after application was sampled and analyzed by Western blotting in an attempt to detect the FT protein. To ensure long-term application to leaves, concentration studies were conducted for EDTA. As a result, 100 mM EDTA applied to the onion epidermis was too high and was found to cause damage to leaves. Then, after further concentration studies, it was confirmed that 1 mM EDTA was able to be used without any problems.

Bud cuttings were prepared from torenia's branches each including the shoot apex. These branches were cut to an even length of approximately 10 cm containing three nodes or so, each of which was put into a 5 ml tube containing 4 ml of 0.1 × PBS, followed by covering the mouth of each tube with a parafilm to avoid evaporation of PBS. Four bud cuttings were prepared for each test group, and test solutions (90 µl each) prepared under three conditions (Condition 1: 0.1 × PBS, 1 mM EDTA, 0.005% Silwet L-77; Condition 2: 33 µg/ml FT protein, 0.1 × PBS, 1 mM EDTA, 0.005% Silwet L-77; Condition 3: FT protein, 20 µM R9 peptide, 0.1 × PBS, 1 mM EDTA, 0.005% Silwet L-77) were applied by being spread thinly over leaves at a position which was two nodes (about 3 cm) apart from the shoot apex. After air drying, these samples were maintained at 25°C in a thermostatic incubator.

After 3 days, the test solutions were applied again, and after one week of the first application, the shoot apexes were sampled. Proteins were extracted from the shoot apex samples by the acetone method. 250 µl of an extraction buffer (10 mM Tris-HCl (pH 8.0), 0.1% CHAPS, 4.5 mM NaCl, 0.1 mM DTT, 0.2 mM EDTA, cOmplete ULTRA 1 tablets (Roche Diagnostics)) was added to each shoot apex sample obtained from 4 bud cuttings, followed by sufficient homogenization in a mixer together with zirconia beads of 4 mm diameter. After centrifugation at 15000 rpm at room temperature for 10 minutes, the supernatant was diluted with 4 volumes of acetone and mixed well, and then allowed to stand on ice for 30 minutes. After further centrifugation at 15000 rpm at 4°C for 30 minutes, the supernatant was removed and the pellet was air-dried for 5 minutes. The pellet was diluted with 50 µl of 1 × PBS and suspended well by pipetting, followed by addition of an equal volume of 2 × sample buffer for protein electrophoresis (concentration: 4% SDS, 20% glycerol, 10% 2-mercaptoethanol, 0.004% bromophenyl blue, 0.125 M Tris-HCl pH 6.8) and thermal treatment at 100°C for 15 minutes. After centrifugation at 15000 rpm at room temperature for 15 minutes, the supernatant was obtained and analyzed in a standard manner by SDS-PAGE and Western blotting in an attempt to detect the FT protein. The primary antibodies used were anti-FT antibody (Santa Cruz Biotechnology) and anti-GFP antibody (Nacalai Tesque, Inc., Japan), and detection was conducted using an ECL PRIME Western Blotting Detection System (Amersham).

As a result of Western blotting, upon combination of the FT protein and the R9 peptide, FT protein signals were able to be detected in the shoot apex (Figure 3). Figure 3 shows the results of Western blotting, in which Lane (1) represents standard FT protein (positive control), and Lanes (2) to (4) represent the shoot apex samples from whole plants treated under Conditions 1 to 3, respectively. This finding confirmed that the applied FT protein migrated from the extracellular environment into cells in leaves and then migrated to the shoot apex.

### [Example 4] Induction of flower buds

In most cases, *in vitro* shoots of torenia will form no flower buds in test tubes. Likewise, *in vitro* shoots of petunia will form no flower buds under short-day conditions. In these *in vitro* shoots, the FT protein was applied to their leaf surface in an attempt to induce flower buds.

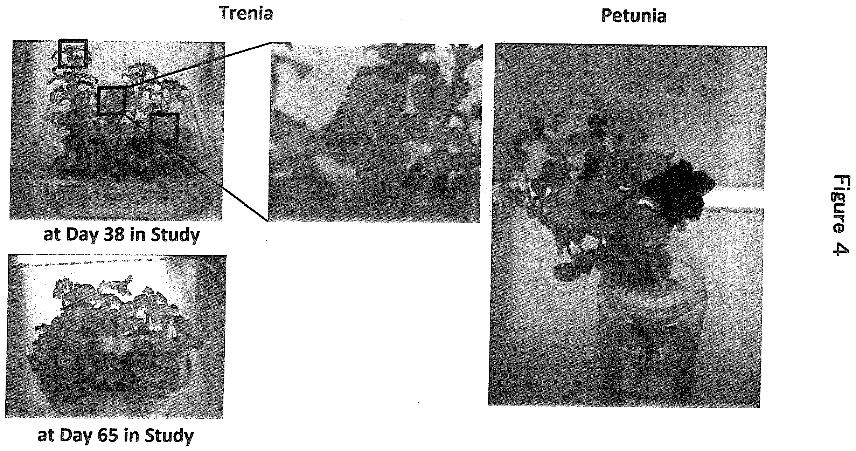

To the shoot apex and leaf surface of torenia and petunia cultivated in test tubes, a test solution (33 µg/ml FT protein, 20 µM R9 peptide, 0.1 × PBS, 1 mM EDTA, 0.005% Silwet L-77) was applied by being spread thinly to ensure a uniform distribution. Application was repeated five times every 2 to 3 days, and culture was also continued thereafter. As a result, flower buds were formed at around 40 days after application and eventually brought into flower in test tubes (Figure 4). This finding indicated that the techniques of the present invention allowed trait modification in plants.

### INDUSTRIAL APPLICABILITY

The method of the present invention can cause phenotypic changes in host plants without changing their genetic traits. Moreover, because of being free from any gene recombination step, the method of the present invention can cause phenotypic changes in host plants in a simple manner. Furthermore, whole plants into which a desired substance has been introduced by the method of the present invention will recover their wild-type phenotypes upon decomposition of the desired substance; and hence the method of the present invention has a smaller impact on the ecosystem and can be regarded as being highly safe for the environment.

### Sequence Listing Free Text

SEQ ID NOs: 1 to 3: synthetic constructs

## Claims

1. A treating agent for plant cell walls, which allows introduction of a desired substance into plant cells, wherein said treating agent comprises a chelator, a surfactant and a cell penetrating peptide.

2. The treating agent according to claim 1, wherein the chelator is a calcium chelator.

3. The treating agent according to claim 1 or 2, wherein the surfactant is a nonionic surfactant.

4. The treating agent according to any one of claims 1 to 3, wherein the cell penetrating peptide is an arginine-rich peptide.

5. A substance delivery method for introduction of a desired substance into a whole plant, which comprises the steps of:
(a) applying the treating agent according to any one of claims 1 to 4 to the surface of the whole plant; and
(b) applying the desired substance to the site where the treating agent has been applied, thereby introducing the desired substance into plant cells.

6. The method according to claim 5, wherein the desired substance has a molecular weight of 1 to 100 kDa.

7. The method according to claim 5 or 6, wherein the cell penetrating peptide is an arginine-rich peptide.

8. The method according to any one of claims 5 to 7, wherein the steps (a) and (b) are carried out at the same time.

9. The treating agent according to any one of claims 1 to 4 or the method according to any one of claims 5 to 8, wherein the desired substance is a macromolecule selected from the group consisting of sugars, proteins, nucleic acids and lipids.

10. The treating agent or the method according to claim 9, wherein the desired substance is a protein.

## Patentansprüche

1. Behandlungsmittel für Pflanzenzellwände, das das Einführen eines gewünschten Stoffes in Pflanzenzellen ermöglicht, wobei das Behandlungsmittel einen Chelator, einen oberflächenaktiven Stoff und ein in Zellen eindringendes Peptid umfasst.

2. Behandlungsmittel nach Anspruch 1, wobei der Chelator ein Calcium-Chelator ist.

3. Behandlungsmittel nach Anspruch 1 oder 2, wobei der oberflächenaktive Stoff ein nichtionischer oberflächenaktiver Stoff ist.

4. Behandlungsmittel nach einem der Ansprüche 1 bis 3, wobei das in Zellen eindringende Peptid ein argininreiches Peptid ist.

5. Stoffzufuhrverfahren zum Einführen eines gewünschten Stoffes in eine ganze Pflanze, das die folgenden Schritte umfasst:
(a) Anwenden des Behandlungsmittels nach einem der Ansprüche 1 bis 4 auf die Oberfläche der ganzen Pflanze; und
(b) Anwenden des gewünschten Stoffes auf die Stelle, auf der das Behandlungsmittel angewendet wurde, wodurch der gewünschte Stoff in Pflanzenzellen eingeführt wird.

6. Verfahren nach Anspruch 5, wobei der gewünschte Stoff ein Molekulargewicht von 1 bis 100 kDa aufweist.

7. Verfahren nach Anspruch 5 oder 6, wobei das in Zellen eindringende Peptid ein argininreiches Peptid ist.

8. Verfahren nach einem der Ansprüche 5 bis 7, wobei die Schritte (a) und (b) gleichzeitig durchgeführt werden.

9. Behandlungsmittel nach einem der Ansprüche 1 bis 4 oder Verfahren nach einem der Ansprüche 5 bis 8, wobei der gewünschte Stoff ein Makromolekül ist, das ausgewählt ist aus der Gruppe bestehend aus Zuckern, Proteinen, Nucleinsäuren und Lipiden.

10. Behandlungsmittel oder Verfahren nach Anspruch 9, wobei der gewünschte Stoff ein Protein ist.

## Revendications

1. Agent de traitement pour des parois de cellules végétales, qui permet l'introduction d'une substance souhaitée dans des cellules végétales, où ledit agent de traitement comprend un chélateur, un tensioactif et un peptide pénétrant les cellules.

2. Agent de traitement selon la revendication 1, dans lequel le chélateur est un chélateur du calcium.

3. Agent de traitement selon la revendication 1 ou 2, dans lequel le tensioactif est un tensioactif non ionique.

4. Agent de traitement selon l'une quelconque des revendications 1 à 3, dans lequel le peptide pénétrant les cellules est un peptide riche en arginine.

5. Procédé de délivrance d'une substance pour l'introduction d'une substance souhaitée dans un plante entière, qui comprend les étapes consistant à :
(a) appliquer l'agent de traitement selon l'une quelconque des revendications 1 à 4 sur la surface de la plante entière ; et
(b) appliquer la substance souhaitée sur le site où l'agent de traitement a été appliqué, en introduisant ainsi la substance souhaitée dans les cellules végétales.

6. Procédé selon la revendication 5, dans lequel la substance souhaitée possède un poids moléculaire de 1 à 100 kDa.

7. Procédé selon la revendication 5 ou 6, dans lequel le peptide pénétrant les cellules est un peptide riche en arginine.

8. Procédé selon l'une quelconque des revendications 5 à 7, dans lequel les étapes (a) et (b) sont réalisées en même temps.

9. Agent de traitement selon l'une quelconque des revendications 1 à 4 ou procédé selon l'une quelconque des revendications 5 à 8, où la substance souhaitée est une macromolécule sélectionnée dans le groupe consistant en des sucres, des protéines, des acides nucléiques et des lipides.

10. Agent de traitement ou procédé selon la revendication 9, dans lequel la substance souhaitée est une protéine.
